# EUROPEAN PATENT SPECIFICATION

(11) **EP 1 501 559 B1**
(45) Date of publication and mention of the grant of the patent: **13.06.2007**
(21) Application number: 03747449.1
(22) Date of filing: 30.04.2003
(51) Int. Cl.: A61L 17/04, A61L 17/06, A61L 17/12

(54) **SURGICAL THREAD AND SURGICAL IMPLANT WITH THE SAME**
CHIRURGISCHER FADEN UND CHIRURGISCHES IMPLANTAT MIT DIESEM FADEN
FIL CHIRURGICAL ET IMPLANT CHIRURGICAL DOTE DE CE DERNIER

(30) Priority: 03.05.2002 DE 10219860
(43) Date of publication of application: 02.02.2005
(73) Proprietor: Ethicon GmbH, 22851 Norderstedt (DE)
(72) Inventor: SCHULDT-HEMPE, Barbara, 24576 Bad Bramstedt (DE); WALTHER, Christoph, 24568 Kattendorf (DE)
(74) Representative: Mercer, Christopher Paul
(86) International application number: PCT/EP2003/004566
(87) International publication number: WO 2003/092758

(56) References cited:
- EP-A- 0 797 962
- EP-A- 0 878 205
- WO-A-01/12073
- US-A- 5 697 969
- US-A- 6 045 571

## Description

The invention relates to a surgical thread as well as a surgical implant which contains such a surgical thread.

Numerous surgical threads and suture materials as well as implants prepared therefrom are known. The threads can be monofilament or mutilfilament as well as resorbable or non-resorbable. See, for example, US 6 045 571 which discloses a thread with an interlocking multifilament sheath and an interlocked multifilament core.

A typical elongation behaviour of a surgical thread under tensile stress is described in US 5 147 382 A using as example a non-resorbable monofilament and illustrated using a stress/strain diagram. The tensile force acting on a thread of given cross-sectional area is plotted as a function of the thereby-effected strain (elongation of the thread, relative to the original length). In the case of low tensile forces, the thread is in the linear-elastic range; no damage occurs as yet and the thread is not permanently deformed. At the end of the linear-elastic range, a permanent deformation of the thread begins at a certain elongation ("yield elongation"). As the tensile force grows, the elongation increases rapidly (visco-elastic range). At the end of the visco-elastic range, the tensile force must rise steeply in order to effect a further elongation of the thread. When, finally, the elongation at break is reached, the thread tears.

For suture materials such as polypropylene or polyamide the linear-elastic range typically extends to an elongation of approx. 1% to 2%. For particularly elastic suture materials, as described in US 5 147 382 A, the linear-elastic range extends to an elongation of approx. 2% to 9%. The visco-elastic range typically ends at an elongation of, e.g., 9% for polypropylene or polyamide and approx. 10% to 30% for particularly elastic suture materials.

Other thread materials, such as, e.g., the silicone elastomer disclosed in US 5 895 413 A, show a visco-elastic elongation range of over 50%. Such materials are elastomeric.

Uses beyond the visco-elastic range are not considered in surgery, as the forces required for a further elongation are too great and in addition cause permanent damage to the thread material.

EP 0 485 986 A1 shows a partly-resorbable composite yarn with a non-resorbable elastic centre (core) which is surrounded by a braided wrapping made of a resorbable and relatively non-elastic yarn component. While the centre endows the yarn with the desired elasticity, the covering provides an additional reinforcement. A similar yarn (with a wound wrapping) is known from EP 0 397 500 B1.

There are application cases, e.g. for special wounds in special tissue or for a special healing process, in which the possibility of a particularly great elongation of the thread is desirable in later healing phases, without excessive forces being necessary. The conventional suture materials mentioned do not have the desired properties, as considerable forces are often necessary for an elongation up to the end of the visco-elastic range, and elongations of clearly over 50% cannot be achieved in practice.

It is the object of the invention to provide a surgical thread (as well as a surgical implant with such a thread), the elongation behaviour of which with respect to tensile forces is adapted to the wound healing process.

This object is achieved by a surgical thread with the features of claim 1, a surgical thread with the features of claim 9 as well as a surgical implant with the features of claim 12. Advantageous versions of the invention result from the dependent claims.

The surgical thread according to the invention has a first component made of resorbable material and a second component made of non-resorbable material and/or slowly resorbable material (which is more slowly resorbable than the material of the first component).. The second component is arranged in the thread in a non-linear manner. It is dimensionally stabilized against tensile forces by the arrangement of the first component, before the resorption of the first component. After the resorption of the first component, this dimensional stabilization is missing, so that, when subjected to a tensile force, the second component can move from its non-linear arrangement into a thereabouts linear arrangement, which is associated with a considerable elongation.

Thus, the two components of the surgical thread are arranged together so that the non- or only slowly resorbable thread portion experiences a more or less marked deflection. This deflection (e.g. in the form of a helical spring or meander-shaped) is fixed in the thread by the first component before the resorption of the first component and remains after the resorption of the first component, at least so long as no tensile forces are acting on the thread. In order to achieve some degree of dimensional stability for the period after the resorption of the first component, the thread, e.g., can be thermally treated during preparation (e.g. between 80°C and 140°C under dry inert gas for approx. 10 hours). As a rule the desired dimensional stability already results from the thread memory occurring during processing, i.e. the second component tends to retain its original non-linear arrangement and essentially reverts to its original position after a change in its arrangement.

In general, after the resorption of the first component a small force is sufficient to considerably stretch the remaining thread. If this elongation has finally led to a linear arrangement of the second component (thus if the remaining thread is "pulled straight"), the material properties of the second component become noticeable and determine the further elongation behaviour under tensile force; in the stress/strain diagram, the linear-elastic range and the visco-elastic range will now be covered as the tensile force rises, as explained above.

The elongation behaviour of the surgical thread according to the invention under a tensile force can be defined within wide limits by the construction of the thread and the arrangement and material properties of the first component and the second component. A linear-elastic range of approx. 1% to 2% is advantageous before the resorption of the first component, which can be achieved, e.g., using a first component arranged as monofilament or multifilament core or by a braided or twisted construction. After the resorption of the first component, the remaining second component shows a changed elongation behaviour which results from the construction (i.e., the arrangement of the second component and the possibilities for a change in length until a linear arrangement is reached) and the preparation conditions (thermal treatment, thread memory, see above) and their influence on the reset behaviour. There are numerous possibilities for determining this behaviour and influencing it within wide limits, as explained in more detail in the following by means of embodiments.

So long as, after a surgical procedure, the first component of the surgical thread according to the invention (or a surgical implant with such a thread) is still not resorbed, the thread has only a low elasticity, like conventional suture material, and therefore ensures a reliable stabilization in the wound area. The elongation behaviour changes during the resorption of the first component; because of the reduction in the material available overall for the absorption of forces, the breaking strength of the thread also decreases as a rule. After the resorption of the first component the remaining thread can be greatly stretched under the action of small forces. The wound scar can therefore yield without problems and is not constricted, which counteracts necrotizing tendencies. If the second component in the remaining thread is aligned in a linear manner, however, considerable forces are necessary for further elongation, i.e. the remaining thread can then absorb forces (up to the force at rupture) in order to prevent damage in the scar area.

There are numerous possibilities for defining a desired curve pattern in the stress/strain diagram through the arrangement of the first component and the second component in the surgical thread.

In a preferred version of the surgical thread according to the invention at least part of the first component is formed as a core which is surrounded by material of the second component. The core can, e.g., be monofilament, but also plied. So long as the essentially linearly aligned core is present, it ensures that the thread stretches like a conventional thread under a tensile force, i.e. relatively little when the forces are small. After the resorption of the core the second component determines the elongation behaviour. If, e.g., the thread is designed as single covered twist yarn, the remaining thread can stretch markedly after the resorption of the core (depending on the helix angle of the wrapping), until it is pulled straight. In the case of a spinning covering twist, the resorption of the first component in the inside of the covering leads to the formation of a cavity which likewise makes possible a relatively marked elongation of the remaining thread. Examples of this are given below.

In another version the thread is designed as plied loop twist with holding threads made of material of the first component and a loop made of material of the second component. In this case as well, the arrangement of the loop, after the resorption of the holding threads, can permit a great elongation of the remaining thread.

The thread can be designed very generally as plied or cabled yarn. Filaments of the first component and the second component are preferably twisted together. During the resorption of the first component a free space forms which makes it possible for the second component to stretch to a high degree under the action of a tensile force. Here also the desired properties of the thread can be defined over a wide range, e.g. via the thickness of the filaments or the twisting (turns per unit of length).

Numerous versions result if the thread comprises a braiding made of material of the first component and of material of the second component (with or without core). The elongation at which the second component is "pulled straight" after the resorption of the first component can be influenced for example via the braiding angle. Some examples are given below for a braided thread.

In another version the thread has a crocheted galloon fabric made of material of the first component and of material of the second component. If the crocheted galloon fabric is relatively narrow, as is the case, e.g., for two fringes made of resorbable material which are connected with a non-resorbable part-weft, such a crocheted galloon fabric can also be regarded as a thread within the meaning of the invention.

Basically, monofilament and/or multifilament components can be used for all preparation, techniques, and the thread thickness can also be varied over a wide range.

In a preferred version of the surgical thread according to the invention, after resorption of the first component, the force required for the linear alignment of the second component is smaller than 5 N. The elongation that occurs should be greater than 1% and can, depending on the construction of the thread, even reach values of well over 100%, as illustrated below using examples.

In an alternative version the surgical thread according to the invention has a first component made of resorbable material and a second component made of non-resorbable material and/or slowly resorbable material which is more slowly resorbable than the material of the first component. Here, however, the second component is arranged in a linear manner in the thread, but highly elastic. Before the resorption of the first component, the second component is dimensionally stabilized against tensile forces by the arrangement of the first component. After the resorption it is already arranged in a linear manner, but, as it is highly elastic and the stabilization by the first component is missing, it can also stretch relatively markedly under the action of small tensile forces. This variant of the invention likewise achieves the above stated object and is based on the same principle. It is explained further below by an embodiment.

Suitable as material for the first component are, in particular, poly-p-dioxanone (PDS), copolymers of glycolide and lactide, preferably in the ratio 90:10 (marketed by Ethicon under the name "Vicryl") and 5:95 (marketed by Ethicon under the name "Panacryl"), pre- degraded copolymers made of glycolide and lactide, preferably in the ratio 90:10 (e.g. "Vicryl", pre-degraded by immersion into a hydrolysis buffer, marketed by Ethicon under the name "Vicryl rapid") as well as copolymers of glycolide and ε-caprolacton (marketed by Ethicon under the name "Monocryl"). However, other resorbable materials or composites from the named or other materials are also conceivable.

The second component can comprise, not only non-resorbable material, but also resorbable material, which, however, is more slowly resorbable than the material of the first component. Preferred materials for the second component are, in the case of resorbable materials, once again poly-p-dioxanone, copolymers made of glycolide and lactide, preferably in the ratio 90:10 and 5:95 as well as copolymers made of glycolide and ε-caprolacton. In the case of the non-resorbable materials, polyamides, polypropylene (marketed by Ethicon under the name "Prolene"), polyesters (marketed by Ethicon under the name "Mersilene") and fluoropolymers are to be mentioned in particular, but other materials, also suitable mixtures and copolymers, are also conceivable. Particularly suitable fluoropolymers are mixtures of polyvinylidene fluoride and copolymers of vinylidene fluoride and hexafluoropropene (marketed by Ethicon under the name "Pronova").

Surgical implants which contain a surgical thread according to the invention can be designed in numerous ways, e.g. as tapes, cords, meshes, mesh strips, tubular implants or three-dimensional constructs. Three-dimensional constructs can be used, e.g., for filling cavities in the tissue or to colonize cells.

Depending on the medical application, the elongation behaviour determined by the thread material of the implant can be advantageous.

In a preferred version the implant has a mesh-like basic shape, in which the ratio of the elongations in two pre-set different directions before the resorption of the first component is different from that after the resorption of the first component. In other words, if a pre-set force per centimeter of implant width acts in one of the directions, the mesh-like basic shape stretches more than if the same force per centimeter of implant width is exerted in the other direction. An implant with these features can be prepared, e.g., from two different threads with different elongation properties so that the elongation properties of the mesh-like basic shape, after the resorption of the first component, are different in longitudinal direction and in transverse direction. Such an implant can be used, e.g., in the surgery of inguinal hernias.

It is advantageous if the force per centimeter of implant width necessary for an elongation of 5% of the implant in a pre-set direction, after the resorption of the first component, is less than 2 N.

The invention is explained further in the following, using embodiments. The figures show in
- Figure 1: a schematic representation of a surgical thread according to the invention designed as a single covered twist yarn according to Example 1,
- Figure 2: a graphical representation of the twist contraction (%) as a function of the number of turns per unit of length (T/m) in the case of single covered twist yarns according to Example 1,
- Figure 3: a schematic representation of a surgical thread according to the invention designed as a spinning covering twist according to Example 2,
- Figure 4: a schematic representation of a surgical thread according to the invention designed as a two-fold twisted yarn according to Example 3,
- Figure 5: a schematic representation of a surgical thread according to the invention designed as a plied loop twist according to Example 4,
- Figure 6: a schematic representation of a surgical thread according to the invention designed as braiding according to Example 5,
- Figure 7: a graphical representation of the force at rupture (in N; dashed curve) as well as the elongation (strain) at break (the numerical value on the ordinate must be multiplied by 10 in order to obtain a figure in %, relative to the initial length; solid line), as a function of the incubation time in a buffer solution (in h) for a surgical thread according to Example 5,
- Figure 8: a schematic representation of a surgical thread according to the invention made using a crochet galloon technique according to Example 7,
- Figure 9: a schematic representation of a surgical thread according to the invention designed as a spinning covering twist with a highly elastic non-resorbable core according to Example 8, and
- Figure 10: a schematic representation of a surgical implant mesh according to the invention.

### Example 1

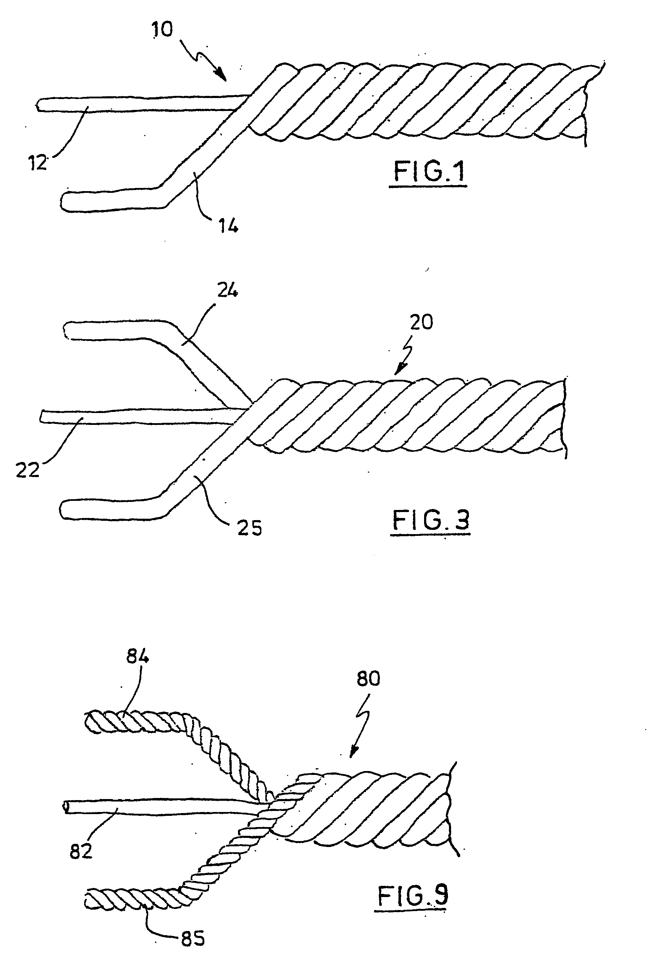

Figure 1 illustrates a single covered twist yarn (covering twist) 10, in which a rectilinearly arranged core 12 of resorbable material is wrapped in a non-resorbable enveloping thread 14.

In the embodiment a monofilament (or in a variant two plied monofilaments) made of a copolymer made of glycolide and ε-caprolacton ("Monocryl", Ethicon) serving as core 12 was wrapped in a monofilament made of a mixture of polyvinylidene fluoride and a copolymer made of vinylidene fluoride and hexafluoropropene ("Pronova", Ethicon) serving as enveloping thread 14, on a Ratti multiple twisting machine. The core 12 was drawn through a hollow spindle and wrapped in the enveloping thread 14 in Z-twist.

In this example a twist contraction of 17% was achieved at a setting of 876 T/m (i.e. turns per meter of thread length) in Z-twist. In other words, the enveloping thread 14 experienced a 17% reduction in length (measured in the direction of the core 12) relative to the original length of the enveloping thread 14, because of the spiral non-linear arrangement on the core 12.

After the resorption of the core 12, the enveloping thread 14 can be pulled straight by a relatively small force so that it stretches again from the reduced length of 83% in the spiral arrangement back to its original length of 100% in linear arrangement. If the force is further increased, the material properties of the enveloping thread 14 become noticeable, as already mentioned at the start. The elongation behaviour of the covering twist 10 is thus decisively co-determined by the twist contraction.

In Table 1 the twist contraction is given for different variants of a covering twist 10 with a single or two-fold plied core 12 made of "Monocryl" and an enveloping thread 14 made of "Pronova". 1 mil = 0.0254 mm. The twist contraction and thus the elongation behaviour can be defined over a wide range.

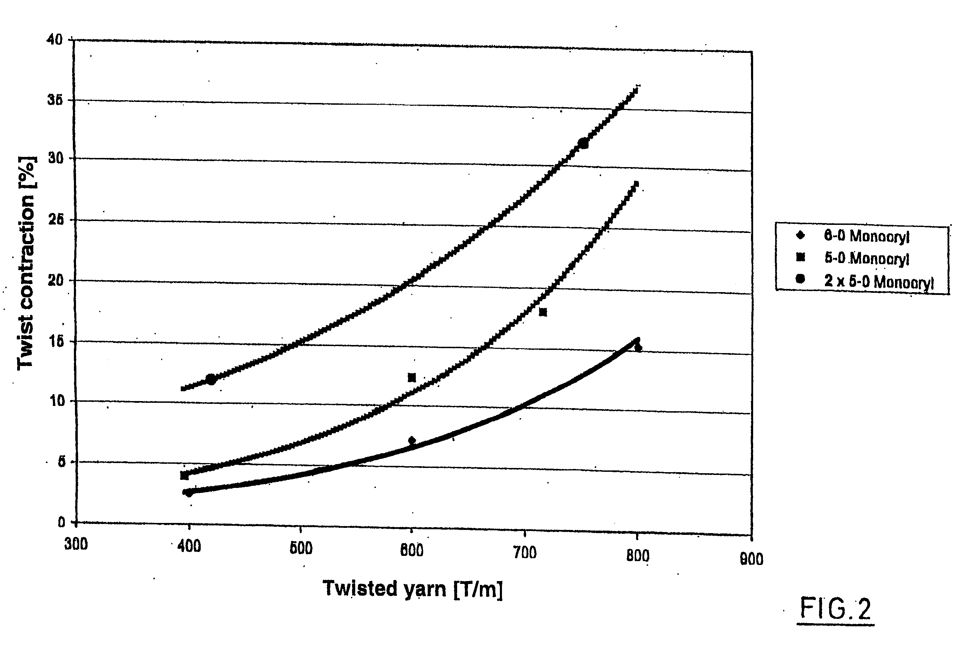

In Figure 2 the twist contraction (in %) is graphically represented for the threads from Table 1 as a function of the set of the twisted thread. In order to illustrate this, a curve is drawn at the corresponding points for each of the three types of cores.

**Table 1: Covering twists of monofilaments**

| **Non** | **Resorbable:** | **Twisted yarn** | **Setting** | **Twist** |
|---|---|---|---|---|
| **resorbable:** | **Monocryl** | | | **contraction** |
| **Pronova** | | | | |
| **Diameter** | **Diameter** | | **[T/m]** | **[%]** |
| **[mil]** | **(acc. to** | | | |
| | **USP)** | | | |
| 3.5 mil | 6-0 | covering twist | 800 Z | 15.3 |
| 3.5 mil | 6-0 | covering twist | 400 Z | 2.6 |
| 3.5 mil | 6-0 | covering twist | 600 Z | 7.2 |
| 3.5 mil | 5-0 | covering twist | 396 Z | 4 |
| 3.5 mil | 5-0 | covering twist | 716 Z | 18 |
| 3.5 mil | 5-0 | covering twist | 600 Z | 12.4 |
| 3.5 mil | 2x5-0 | covering twist | 752 Z | 32 |
| 3.5 mil | 2x5-0 | covering twist | 420 Z | 12 |

### Example 2

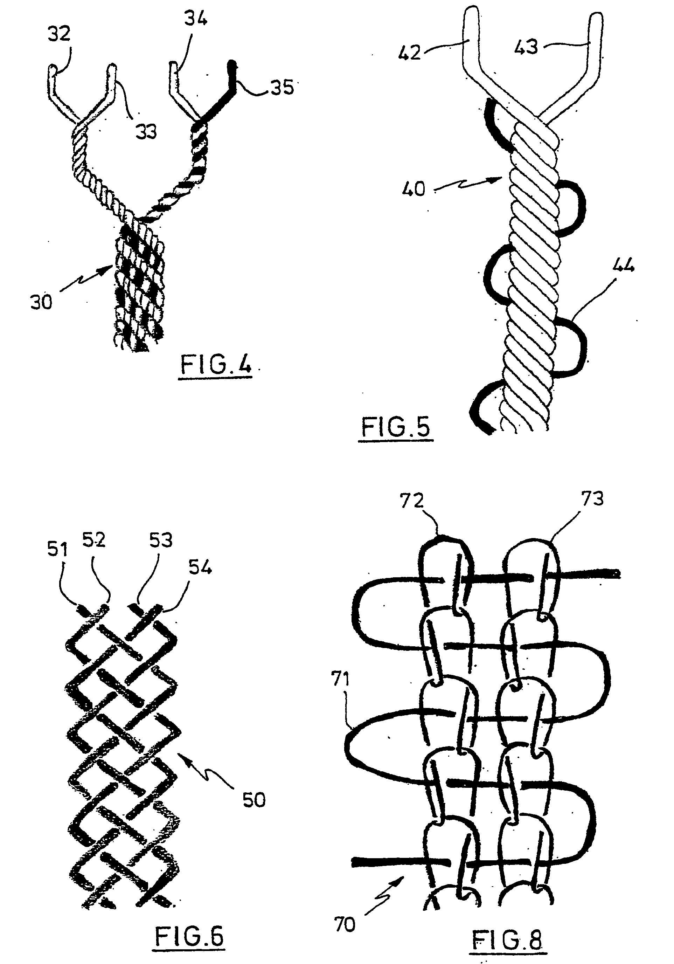

Figure 3 shows a version in which the surgical thread is designed as a spinning covering twist 20 with a core 22 and two oppositely directed enveloping threads 24 and 25. As in Example 1, the core 22 can consist, e.g., of a "Monocryl" monofilament, while, e.g., "Pronova" is used for the enveloping threads 24 and 25.

After the resorption of the core 22 the remaining thread can be stretched under relatively little expenditure of force, because the enveloping threads 24 and 25 can move into the cavity at the site of the core 22. Because the enveloping threads 24 and 25, twisted in opposite directions, are mutually stabilizing, however, this elongation is, as a rule, less than for the covering twist 10 from Example 1.

### Example 3

In Figure 4 a cabled yarn without a core, namely a quadruple double twist 30, is shown as a further version of the thread.

A 1x4-fold twisted yarn was twisted in S- and Z-twist on a Lezzeni TBR P two-stage twisting machine. In each case, a non-resorbable monofilament made of polyamide ("Ethilon", Ethicon) and a rapidly resorbable thread made of a pre-degraded copolymer of glycolide and lactide in the ratio 90:10 ("Vicryl rapid", Ethicon) was used.

The twisted yarn 30 therefore contains a non-resorbable part-thread 32 as well as a resorbable part-thread 33 in the first stage (Z-twist), and a non-resorbable part-thread 34 as well as a resorbable part-thread 35 in the second stage (S-twist). After the resorption of the resorbable part-threads 33 and 35, the remaining thread can be more easily stretched, for similar reasons as in Example 2.

### Example 4

Figure 5 illustrates as a further version a plied loop twist 40 which was prepared using two holding threads 42 and 43 from a resorbable copolymer made of glycolide and lactide in the ratio 90:10 ("Vicryl", Ethicon) and a non-resorbable loop thread 44 made of polypropylene ("Prolene", Ethicon).

After resorption of the holding threads 42 and 43, the loop thread 44 arranged in meander form can be easily pulled straight. In the embodiment a 20% change in length of the loop thread was able to be achieved in this way. In the geometry shown in Figure 5, an even greater change in length results.

### Example 5

In Figure 6 a braiding 50 is schematically represented as a further version of a surgical thread.

In the embodiment the braiding 50 was braided from four part-threads, namely from a monofilament (diameter 0.09 mm) 51 of the non-resorbable material "Pronova" (see above) and from three monofilaments (diameter 0.09 mm) 52, 53, 54 of the resorbable material "Monocryl" (see above), at 48 braids per inch.

The intact braided structure behaves like a conventional thread. After the resorption of the resorbable part-threads 52, 53 and 54, the remaining "Pronova" monofilament 51 can be pulled straight with relatively little force, which leads to a great elongation. In the case of an even greater tensile force the "Pronova" monofilament 51 shows in the stress/strain diagram firstly the linear and then the visco-elastic range, as explained at the start.

In particular in the visco-elastic range, there is a further considerable elongation until the remaining thread consisting of the monofilament 51 tears at the rupture force Fₘₐₓ.

In Table 2 are shown for the braiding 50 prepared in the embodiment for different resorption stages of the "Monocryl" monofilaments 52, 53, 54, the force at rupture Fₘₐₓ, the elongation at rupture, the force necessary to pull straight the remaining thread, the elongation which then occurs as well as the elongation occurring in the case of a force of approx. 2 N exerted on the remaining thread in the linear or visco-elastic range of the pulled-straight remaining thread. All elongations are relative to the original length of the thread if the "Pronova" monofilament 51 is arranged in a non-linear manner. Serving as a measure of the degree of resorption of the "Monocryl" monofilaments 52, 53 and 54 is the incubation time (in hours, h) in a hydrolysis buffer with the pH value 7.26 simulating a physiological situation, which was heated to 50.5°C to accelerate the resorption.

It is recognised that at the beginning (0 hours incubation) the thread has a high rupture force and a relatively low elongation at break. After an incubation time of 24 hours a decrease in the strength of the "Monocryl" monofilaments 52, 53 and 54 as a result of resorption becomes noticeable, and the elongation at break has increased. This effect intensifies after an incubation time of 48 hours. After an incubation time of 72 hours the resorbable component of the thread is already so weakened that the "Pronova" monofilament 51. can be practically pulled straight with a relatively low force of 1 N, which leads to a 100% elongation. The force at rupture is already largely determined by the properties of the "Pronova" monofilament 51. After 96 hours or 120 hours incubation time the remaining thread can be pulled straight with a force of less than 0.5 N. The force at rupture hardly changes compared with an incubation time of 72 hours, while the elongation at break is still increasing.

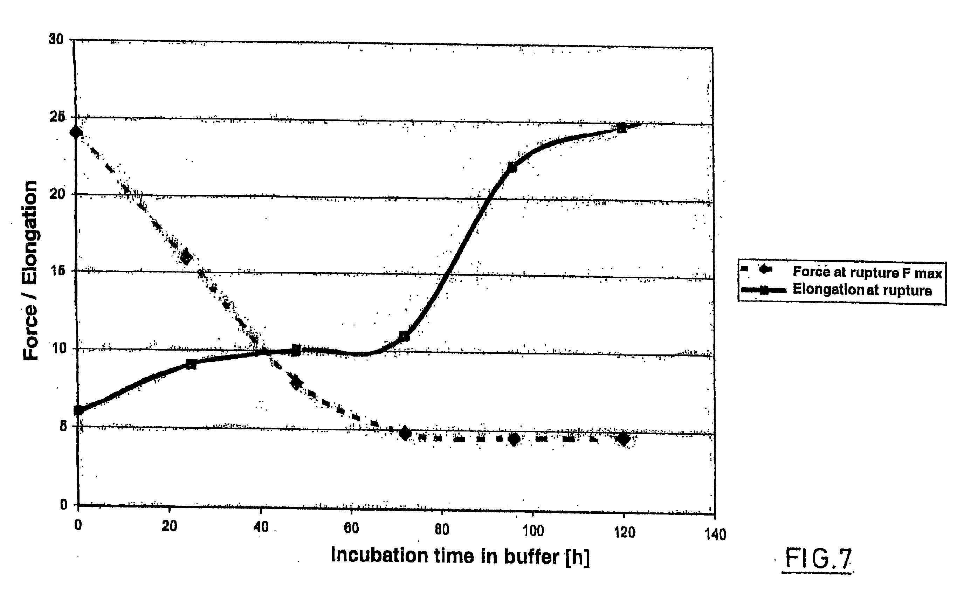

The numerical values from Table 2 are plotted on a graph in Figure 7. The force at rupture (dotted line) is given in N, while the numerical values on the ordinate still have to be multiplied by 10 in order to obtain the elongation at break (solid curve) in percent.

**Table 2: Braided thread made of monofilament part-threads**

| Material | Incubation | Pulling straight | | Linear-visco-elastic range | | Force at rupture Fₘₐₓ | Elongation at break |
|---|---|---|---|---|---|---|---|
| Braid of 1 Pronova monofilament (diameter 3.5 mils, i.e. approx. 0.09 mm) and 3 Monocryl monofilaments (diameter each 3.5 mils), approx. 48 braids per inch | At 50.5°C in a phosphate buffer with pH 7.26 | Force [N] | Elongation [%] | Force [N] | Elongation [%] | [N] | [%] |
| | 0 h | | | | | approx. 24 | 60 |
| | 24 h | | | | | approx. 16 | 90 |
| | 48 h | | | | | 8 | 100 |
| | 72 h | approx. 1 | approx. 100 | approx. 2 | Approx. 105 | 4.8 | > 110 |
| | 96 h | < 0.5 N | approx. 110 | approx. 2 | Approx. 140 | 4.45 | 220 |
| | 120 h | < 0.5 N | approx. 120 | approx. 2 | Approx. 150 | 4.65 | 248 |

### Example 6

Table 3 shows examples of further braided threads which are designated (a) to (j). The braid construction is given in a manner familiar to a person skilled in the art. Polypropylene ("Prolene", Ethicon) was used as non-resorbable component and a copolymerisate of glycolide and lactide in the ratio 90:10 ("Vicryl", Ethicon) was used as resorbable component. Table 3 also shows some numerical values from the stress/strain diagram, namely the elongation at a force of 5 N as well as the force at rupture Fₘₐₓ before the start of resorption (initial) and the elongations at forces of 0.1 N, 1 N and 5 N as well as the force at rupture Fₘₐₓ after an incubation time of 7 days under in-vitro conditions (hydrolysis buffer as in Example 5). The braids were manufactured on a 16-feed head with central core guiding.

**Table 3: Examples of braided threads (braid constructions: 16-feed head with central core guiding**

| | | | | | Bobbin count, non-resorbable portion | Elongation at 5N/ initial | F max/ initial | Elongation at 0.1 N/7d in vitro | Elongation at 1 N/7d in vitro | Elongation at 5 N/7d in vitro | F max/7d in vitro |
|---|---|---|---|---|---|---|---|---|---|---|---|
| Designation | Braiding | Material | | | | [%] | [N] | [%] | [%] | [%] | [N] |
| (a) | 16+3/24 Z | 4 (4x80) den Prolene twisted yarn | 12x56 den Vicryl | Core: 3x56 den Vicryl | 21 + 2r | | 96.7 | 2.2 | 3.6 | 7.5 | 61.9 |
| (b) | 16+3/24Z | 4 (2x70) den Prolene twisted yarn | 12x56 den Vicryl | Core: 3x56 den Vicryl | 21 + 2r | 0.8 | 77.3 | 2.1 | 3.6 | 7.5 | 31.1 |
| (c) | 16+3/24 Z | 4x3.5 mils Prolene monofilament | 12x56 den Vicryl | Core: 3x56 den Vicryl | 2l+2r | 0.8 | 66.6 | 1.2 | 1.9 | 6.8 | 15.5 |
| (d) | 16+3/24 Z | 2x(3-0) Prolene monofilament | 14x56 den Vicryl | Core: 3x56 den Vicryl | 1l+1r | 1.3 | 104.9 | 0.7 | 1.4 | 3.5 | 64.2 |
| (e) | 16+3/32 Z | 8x60 den Prolene | 8x56 den Vicryl | Core: 3x56 den Vicryl | 81 | 0.7 | 65.1 | 1.1 | 2.8 | - | 4.7 |
| (f) | 16+3/22 Z | 8x60 den Prolene | 8x56 den Vicryl | Core: 3x56 den Vicryl | 81 | 0.8 | 64.26 | 1.5 | 2.7 | 13.9 | 11.28 |
| (g) | 16+3/24 Z | 8x60 den Prolene | 8x56 den Vicryl | Core: 3x56 den Vicryl | 4l + 4r | 0.9 | 60.3 | 1.9 | 2.5 | 7.8 | 22.7 |
| (h) | 16+3/24 Z | 4x60 den Prolene | 12x56 den Vicryl | Core: 3x56 den Vicryl | 21 + 2r | 0.9 | 59.7 | 2.3 | 3.6 | 7.9 | 24.3 |
| (i) | 16+3/24 Z | 2×60 den Prolene | 14x56 den Vicryl | Core: 3x56 den Vicryl | 21 | 1 | 54.6 | 1.3 | 3.2 | 8.1 | 24.7 |
| (j) | 16+3/24 Z | 2x60 den Prolene | 14x80 den Vicryl | Core: 3x80 den Vicryl | 21 | 0.9 | 6.0 | 1.8 | 4.3 | 8.4 | 24.6 |

### Example 7

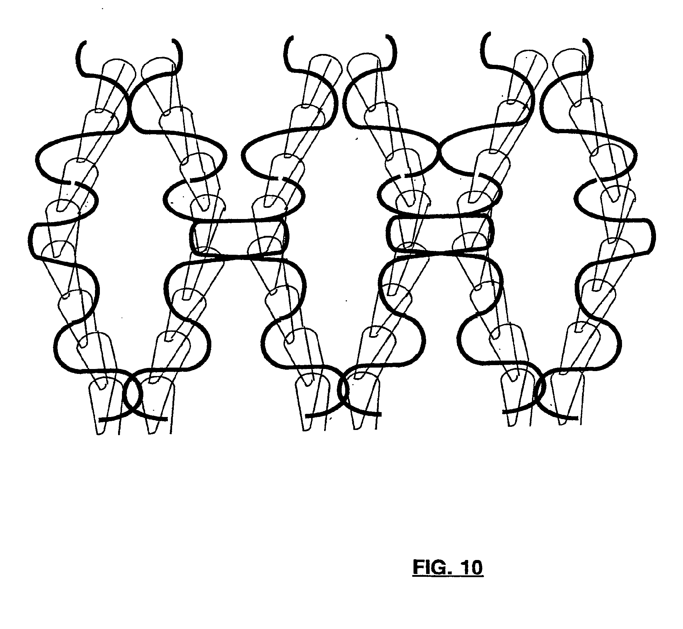

The surgical "thread" 70 represented in Figure 8 was made using a crocheted galloon technique.

To this end, using a Gauge 8 Müller crochet galloon machine, a non-resorbable part-weft 71 was guided, with the bar movement 0/4//, through two resorbable warp threads 72 and 73, in pillar stitch formation.

After resorption of the warp threads 72, 73, the part-weft 71 can be pulled straight with a slight force, which leads to a great elongation.

### Example 8

Figure 9 shows as a further version of a surgical thread with the desired elongation behaviour a spinning covering twist 80, which, contrary to the spinning covering twist from Example 2, contains not a resorbable core, but a core 82 made of a non-resorbable material. The core 82 is highly elastic and wrapped in a protective casing comprising two resorbable enveloping threads 85 and 85.

Before the resorption of the enveloping threads'84 and 85 the elongation behaviour of the spinning covering twist 80 is largely determined by the enveloping threads 84 and 85, so that the thread behaves like a conventional twisted yarn. After the resorption the stabilizing effect of the enveloping threads 84 and 85 is missing so that the highly elastic properties of the core 82 become noticeable. The remaining thread can therefore be strongly stretched using a little force.

In the embodiment the spinning covering twist 80 was made on a multiple twisting machine using a partly-orientated, elastic monofilament made of polypropylene as core 82.

The core 82 was guided through a hollow spindle, and the enveloping threads 84 and 85 were spun round the core. After resorption of the enveloping threads 84 and 85, the core 82 had an elongation at rupture of 70% to 100%.

### Example 9

In order to prepare a surgical implant in the form of a mesh from a surgical thread with the considered elongation behaviour, firstly a lx2-fold twisted yarn at 171 T/m in S-twist and 156 T/m in Z-twist was twisted on a Lezzini TBR P two-stage twisting machine. For this, in each case a polypropylene monofilament ("Prolene", Ethicon) with a diameter of 3.5 mils (1 mil = 0.0254 mm) and a monofilament (# 6-0 according to USP) made of "Monocryl" (see Example 1) was used.

This twisted yarn was processed on a Gauge 6 Gomez crochet galloon machine as a part-weft with the bar movement 0-4/2-4/2-6/2-4/2-4// to give a light-weight partly resorbable mesh.

Finally, the obtained mesh was thermally fixed at a temperature between 80°C and 140°C under dry inert gas for approx. 10 hours. The consequence of such a thermal fixing, which is familiar to a person skilled in the art, is that the component made of non-resorbable material largely retains its shape after the resorption of the resorbable component (thread memory effect). The forces necessary for the stretching are, however, much smaller as the dimensional stabilization by the resorbable component is missing, which leads to the desired elongation properties, as explained. Comparable fixing conditions are also suitable for the surgical threads according to Examples 1 to 8.

### Example 10

A warp-knitted implant mesh was prepared as described in Example 9, the double twists used having different colours, e.g. using "Prolene", blue (Ethicon) combined with "Monocryl", violet (Ethicon). The mesh was made with the warping ratio of 6 threads (undyed) to 2 threads (dyed).

### Example 11

Figure 10 displays a schematic representation of another implant mesh containing surgical threads with the considered elongation behaviour. All embodiments of this mesh were produced in crochet-galloon technique on a Gauge 8 "Müller-Raschelina" chrochet galloon maschine. The mesh pattern is defined by:

| | |
|---|---|
| Warp: closed pillar stitch | 1-0// |
| Part-weft I: | 6-2/4-0/4-2// |
| Part-weft II: | 4-0/4-2/6-2// |

Individual embodiments were made by using different kinds of threads:

### Embodiment 1:

Warp: polypropylen monofilament 3.5 mils (1 mil = 0.0254 mm) ("Prolene", Ethicon);
Part-wefts I and II: thread as described by means of Figure 1 (Example 1), i.e. single covered twist yarn.

### Embodiment 2:

Warp: thread as described by means of Figure 4 (Example 3), i.e. 1x4-fold twisted yarn in S- and Z-twist;
Part-wefts I and II: thread as described by means of Figure 4 (Example 3), i.e. 1x4-fold twisted yarn in S- and Z-twist.

### Embodiment 3:

Warp: thread as described by means of Figure 9 (Example 8), i.e. spinning covering twist;
Part-wefts I and II: thread as described by means of Figure 5 (Example 4), i.e. plied loop twist.

## Claims

1. A surgical thread comprising a core (12, 22, 82) that is made from at least one resorbable material and a covering (10, 20, 80) that is made from at least one non-resorbable material and/or slowly resorbable material which is more slowly resorbable than the resorbable material of the core, the covering comprising threads and **characterised in that** the threads of the covering are arranged in the surgical thread as a single covered twist or a spinning covering twist and wherein before the resorption of the core, the threads of the covering are dimensionally stabilized against tensile forces by the core, and wherein after resorption of the core, the dimensional stabilisation is missing so that when subjected to a tensile force, the covering can move from its non-linear arrangement into a thereabouts linear arrangement.

2. A surgical thread comprising holding threads (42, 43) made from at least one resorbable material and a loop (44) made from at least one non-resorbable and/or slowly resorbable material, in a plied loop twist (40), **characterised in that**, before resorption of the holding threads, the loop is dimensionally stabilised against tensile forces by the holding threads, and wherein after resorption of the holding threads, the dimensional stabilisation is missing so that when subjected to a tensile force, the loop can move from its non-linear arrangement into a thereabouts linear arrangement.

3. A surgical thread that is designed as a plied or cabled yarn, comprising at least two yarns that are twisted about an axis, each of the two yarns comprising at least one resorbable thread (33, 35) and at least one non-resorbable and/or slowly resorbable thread (32, 34), **characterised in that**, before resorption of the at least one resorbable thread, the at least one non-resorbable and/or slowly resorbable thread is dimensionally stabilised against tensile forces by the at least one resorbable thread, and wherein after resorption of the at least one resorbable thread, the dimensional stabilisation is missing so that when subjected to a tensile force, the at least one non-resorbable and/or slowly resorbable thread can move from its non-linear arrangement into a thereabouts linear arrangement.

4. A braided surgical thread comprising at least one resorbable thread (52, 53, 54) and at least two non-resorbable and/or slowly resorbable threads (51), **characterised in that**, before resorption of the at least one resorbable thread, the at least two non-resorbable and/or slowly resorbable threads is dimensionally stabilised against tensile forces by the at least one resorbable thread, and wherein after resorption of the at least one resorbable thread, the dimensional stabilisation is missing so that when subjected to a tensile force, the at least two non-resorbable and/or slowly resorbable threads can move from its non-linear arrangement into a thereabouts linear arrangement.

5. A surgical thread that is made using a crochet galloon technique, comprising at least one warp thread (72, 73) made from at least one resorbable material and a weft thread (71) made from at least one non-resorbable and/or slowly resorbable material, **characterised in that**, before resorption of the warp thread, the weft thread is dimensionally stabilised against tensile forces by the warp thread, and wherein after resorption of the warp thread, the dimensional stabilisation is missing so that when subjected to a tensile force, the weft thread can move from its non-linear arrangement into a thereabouts linear arrangement.

6. The surgical thread according to any preceding claim, wherein, after resorption of the at least one resorbable material, the force required for the linear alignment of the non-resorbable and/or slowly resorbable material is smaller than 5 N.

7. The surgical thread according to any one of claims 1 to 6 wherein the resorbable material is poly-p-dioxanone, a copolymer of glycolide and lactide, a pre-degraded copolymer of glycolide and lactide or a copolymer of glycolide and ε-caprolactone.

8. The surgical thread according to any one of claims 1 to 7 wherein the non-resorbable and/or slowly resorbable material is poly-p-dioxanone, a copolymer of glycolide and lactide, a copolymer of glycolide and ε-caprolactone, a polyamide, polypropylene, a polyester, a fluoropolymer, a mixture of polyvinylidene fluoride or a copolymer of vinylidene fluoride and hexafluoropropene.

9. A surgical implant comprising at least one surgical thread according to any one of claims 1 to 8.

10. The surgical implant according to claim 9, wherein the implant is a tape, a cord, a mesh, a mesh strip, a tubular implant or a three-dimensional construct.

11. The surgical implant according to claim 10 wherein the force, per cm of implant width, required for an elongation of 5% in a first direction after the resorption of the at least one resorbable material is less than 2 N.

## Patentansprüche

1. Chirurgischer Faden mit einem Kern (12, 22, 82), der aus mindestens einem resorbierbarem Material hergestellt ist, und einer Ummantelung (10, 20, 80), die aus mindestens einem nichtresorbierbarem Material und/oder langsam resorbierbarem Material hergestellt ist, das langsamer resorbierbar als das resorbierbare Material des Kerns ist, wobei die Ummantelung Fäden umfaßt, **dadurch gekennzeichnet, daß** die Fäden der Ummantelung in dem chirurgischen Faden als ein Einfachumwindezwirn oder ein Spinnwindezwirn angeordnet sind und vor der Resorption des Kerns die Fäden durch den Kern gegen Zugkräfte formbeständig sind und daß nach Resorption des Kerns die Formbeständigkeit fehlt, so daß sich die Ummantelung, wenn sie einer Zugkraft ausgesetzt ist, von ihrer nichtlinearen Anordnung in eine ungefähr lineare Anordnung bewegen kann.

2. Chirurgischer Faden mit Haltefäden (42, 43), die aus mindestens einem resorbierbarem Material hergestellt sind, und einem Schlaufenfaden (44), der aus mindestens einem nichtresorbierbarem und/oder langsam resorbierbarem Material hergestellt ist, in einem dublierten Schlaufenzwirn (40), **dadurch gekennzeichnet, daß** vor Resorption der Haltefäden die Schlaufe durch die Haltefäden gegen Zugkräfte formbeständig ist und daß nach Resorption der Haltefäden die Formbeständigkeit fehlt, so daß sich die Schlaufe, wenn sie einer Zugkraft ausgesetzt ist, von ihrer nichtlinearen Anordnung in eine ungefähr lineare Anordnung bewegen kann.

3. Chirurgischer Faden, der als ein dublierter oder mehrstufiger Zwirn gestaltet ist, umfassend mindestens zwei Garne, die um eine Achse gedreht sind, wobei jedes der beiden Garne mindestens einen resorbierbaren Faden (33, 35) und mindestens einen nichtresorbierbaren und/oder langsam resorbierbaren Faden (32, 34) umfaßt, **dadurch gekennzeichnet, daß** vor Resorption des mindestens einen resorbierbaren Fadens der mindestens eine nichtresorbierbare und/oder langsam resorbierbare Faden durch den mindestens einen resorbierbaren Faden gegen Zugkräfte formbeständig ist, und daß nach Resorption des mindestens einen resorbierbaren Faden die Formbeständigkeit fehlt, so daß sich der mindestens eine nichtresorbierbare und/oder langsam resorbierbare Faden, wenn er einer Zugkraft ausgesetzt ist, von seiner nichtlinearen Anordnung in eine ungefähr lineare Anordnung bewegen kann.

4. Geflochtener chirurgischer Faden mit mindestens einem resorbierbaren Faden (52, 53, 54) und mindestens zwei nichtresorbierbaren und/oder langsam resorbierbaren Fäden (51), **dadurch gekennzeichnet, daß** vor Resorption des mindestens einen resorbierbaren Fadens die mindestens zwei nichtresorbierbaren und/oder langsam resorbierbaren Fäden durch den mindestens einen resorbierbaren Faden formbeständig sind und daß nach Resorption des mindestens einen resorbierbaren Fadens die Formbeständigkeit fehlt, so daß sich die mindestens zwei nicht resorbierbaren und/oder langsam resorbierbaren Fäden, wenn sie einer Zugkraft ausgesetzt sind, von ihrer nichtlinearen Anordnung in eine ungefähr lineare Anordnung bewegen können.

5. Chirurgischer Faden, der unter Verwendung einer Häkelgalontechnik hergestellt ist, mit mindestens einem Kettfaden (72, 73), der aus mindestens einem resorbierbarem Material hergestellt ist, und einem Schußfaden (71), der aus mindestens einem nichtresorbierbaren und/oder langsam resorbierbaren Material hergestellt ist, **dadurch gekennzeichnet, daß** vor Resorption des Kettfadens der Schußfaden durch den Kettfaden gegen Zugkräfte formbeständig ist und daß nach Resorption des Kettfadens die Formbeständigkeit fehlt, so daß sich der Schußfaden, wenn er einer Zugkraft ausgesetzt ist, von seiner nichtlinearen Anordnung in eine ungefähr lineare Anordnung bewegen kann.

6. Chirurgischer Faden nach einem der vorangehenden Ansprüche, **dadurch gekennzeichnet, daß** nach Resorption des mindestens einen resorbierbarem Materials die für die lineare Ausrichtung des nichtresorbierbaren und/oder langsam resorbierbaren Materials erforderliche Kraft geringer als 5 N ist.

7. Chirurgischer Faden nach einem der Ansprüche 1 bis 6, **dadurch gekennzeichnet, daß** das resorbierbare Material Poly-p-Dioxanon, ein Copolymer aus Glycolid und Lactid, ein vorabgebautes Copolymer aus Glycolid und Lactid oder ein Copolymer aus Glycolid und ε-Caprolacton ist.

8. Chirurgischer Faden nach einem der Ansprüche 1 bis 7, **dadurch gekennzeichnet, daß** das nichtresorbierbare und/oder langsam resorbierbare Material Poly-p-Dioxanon, ein Copolymer aus Glycolid und Lactid, ein Copolymer aus ε-Caprolacton, ein Polyamid, Polypropylen, ein Polyester, ein Fluorpolymer, eine Mischung aus Polyvinylidenfluorid oder ein Copolymer aus Vinylidenfluorid und Hexafluorpropen ist.

9. Chirurgisches Implantat mit mindestens einem chirurgischen Faden nach einem der Ansprüche 1 bis 8.

10. Chirurgisches Implantat nach Anspruch 9, **dadurch gekennzeichnet, daß** das Implantat ein Streifen, eine Schnur, ein Netz, ein Netzstreifen, ein rohrförmiges Implantat oder ein dreidimensionales Konstrukt ist.

11. Chirurgisches Implantat nach Anspruch 10, **dadurch gekennzeichnet, daß** die Kraft, pro cm Implantatbreite, die für eine Dehnung um 5 % in einer ersten Richtung erforderlich ist, nach der Resorption des mindestens einen resorbierbaren Materials geringer als 2 N ist.

## Revendications

1. Fil chirurgical comprenant une âme (12, 22, 82) qui est réalisée à partir d'au moins un matériau résorbable et un recouvrement (10, 20, 80) qui est réalisé à partir d'au moins un matériau non résorbable et/ou un matériau lentement résorbable qui est résorbable plus lentement que le matériau résorbable de l'âme, le recouvrement comprenant des fils et **caractérisé en ce que** les fils du recouvrement sont agencés dans le fil chirurgical selon une torsion recouverte unique ou une torsion de recouvrement de filature et dans lequel avant la résorption de l'âme, les fils du recouvrement sont dimensionnellement stabilisés contre les forces de traction par l'âme, et dans lequel après la résorption de l'âme, la stabilisation dimensionnelle manque de sorte que lorsqu'il est soumis à une force de traction, le recouvrement peut passer de son agencement non linéaire à un agencement à peu près linéaire.

2. Fil chirurgical comprenant des fils de support (42, 43) réalisés à partir d'au moins un matériau résorbable et une boucle (44) réalisée à partir d'au moins un matériau non résorbable et/ou lentement résorbable, dans un fil retors bouclé (40), **caractérisé en ce qu'**après la résorption des fils de support, la boucle est dimensionnellement stabilisée contre les forces de traction par les fils de support, et dans lequel après la résorption des fils de support, la stabilisation dimensionnelle manque de sorte que lorsqu'elle est soumise à une force de traction, la boucle peut passer de son agencement non linéaire à un agencement à peu près linéaire.

3. Fil chirurgical qui est conçu comme un fil retors ou câblé comprenant au moins deux fils qui sont torsadés autour d'un axe, chacun des deux fils comprenant au moins un fil résorbable (33, 35) et au moins un fil non résorbable et/ou lentement résorbable (32, 34), **caractérisé en ce qu'**après la résorption d'au moins un fil résorbable, le au moins un fil non-résorbable et/ou lentement résorbable qui est dimensionnellement stabilisé contre les forces de traction par le au moins un fil résorbable, et dans lequel après la résorption du au moins un fil résorbable, la stabilisation dimensionnelle manque de sorte que lorsqu'il est soumis à une force de traction, le au moins un fil non-résorbable et/ou lentement résorbable peut passer de son agencement non linéaire à un agencement à peu près linéaire.

4. Fil chirurgical tressé comprenant au moins un fil résorbable (52, 53, 54) et au moins deux fils non résorbables et/ou lentement résorbables (51), **caractérisé en ce que** avant la résorption du au moins un fil résorbable, les au moins deux fils non-résorbables et/ou lentement résorbables sont dimensionnellement stabilisés contre les forces de traction par le au moins un fil résorbable, et dans lequel après la résorption du au moins un fil résorbable, la stabilisation dimensionnelle manque de sorte que lorsqu'ils sont soumis à une force de traction, les au moins deux fils non-résorbables et/ou lentement résorbables peuvent passer de leur agencement non linéaire à un agencement à peu près linéaire.

5. Fil chirurgical qui est réalisé en utilisant une technique à crocheter les galons, comprenant au moins un fil de chaîne (72, 73), réalisé à partir d'au moins un matériau résorbable et un fil de trame (71) réalisé à partir d'au moins un matériau non résorbable et/ou lentement résorbable, **caractérisé en ce qu'**après la résorption du fil de chaîne, le fil de trame est dimensionnellement stabilisé contre les forces de traction par le fil de chaîne, et dans lequel après la résorption du fil de chaîne, la stabilisation dimensionnelle manque de sorte que lorsqu'il est soumis à une force de traction, le fil de trame peut passer de son agencement non linéaire à un agencement à peu près linéaire.

6. Fil chirurgical selon l'une quelconque des revendications précédentes dans lequel après la résorption du au moins un matériau résorbable, la force nécessaire pour l'alignement linéaire du matériau non résorbable et/ou lentement résorbable est inférieure à 5 N.

7. Fil chirurgical selon l'une quelconque des revendications 1 à 6, dans lequel le matériau résorbable est de la poly-p-dioxanone, un copolymère de glycolide et de lactide, un copolymère prédégradé de glycolide et de lactide, ou un copolymère de glycolyde et de ε-caprolactone.

8. Fil chirurgical selon l'une quelconque des revendications 1 à 7, dans lequel le matériau non résorbable et/ou lentement résorbable est de la poly-p-dioxanone, un copolymère de glycolide et de lactide, un copolymère de glycolide et de ε-caprolactone, un polyamide, un polypropylène, un polyester, un polymère fluoré, un mélange de fluorure de polyvinylidène ou un copolymère de fluorure de vinylidène et d'hexafluoropropène.

9. Implant chirurgical comprenant au moins un fil chirurgical selon l'une quelconque des revendications 1 à 8.

10. Implant chirurgical selon la revendication 9, dans lequel l'implant est une bande, un cordon, une maille, une bande de maille, un implant tubulaire ou une construction tridimensionnelle.

11. Implant chirurgical selon la revendication 10, dans lequel la force par cm de largeur d'implant nécessite un arrangement de 5% dans une première direction après que la résorption du au moins un matériau résorbale est inférieure à 2 N.
